# EUROPÄISCHE PATENTSCHRIFT

(11) **EP 1 572 344 B1**
(45) Veröffentlichungstag und Bekanntmachung des Hinweises auf die Patenterteilung: **28.10.2009**
(21) Anmeldenummer: 03813539.8
(22) Anmeldetag: 18.12.2003
(51) Int. Cl.: B01J 13/02, B01J 13/04, A61K 9/50, A23L 1/00

(54) **PELLETS UND VERFAHREN ZU SEINER HERSTELLUNG**
PELLETS AND METHOD FOR PRODUCTION THEREOF
GRANULES ET PROCEDE DE PRODUCTION DESDITS GRANULES

(30) Priorität: 19.12.2002 DE 10260658; 28.02.2003 DE 10309777
(43) Veröffentlichungstag der Anmeldung: 14.09.2005
(73) Patentinhaber: IPC Process Center GmbH & Co., 01277 Dresden (DE)
(72) Erfinder: KEMPE, Wolfgang, 01169 Dresden (DE); MINOR, Marcel, NL-6704 AA Wageningen (NL); ADAMSE, Marijke, NL-6716 GN Ede (NL)
(74) Vertreter: Pfenning, Meinig & Partner GbR
(86) Internationale Anmeldenummer: PCT/DE2003/004232
(87) Internationale Veröffentlichungsnummer: WO 2004/056469

(56) Entgegenhaltungen:
- EP-A- 0 598 920
- EP-A- 1 214 892
- EP-A- 1 252 831
- WO-A-03/018186

## Beschreibung

Die Erfindung betrifft Pellets, die mindestens eine ölige Komponente enthalten, die für sich gesehen allein eine aktive Komponente darstellt oder mindestens eine solche aktive Komponente enthält.

In der Regel sind solche öligen und/oder aktiven Komponenten häufig oxidationsempfindlich oder leicht flüchtig, so dass über mehr oder weniger große Zeiträume der Anteil solcher aktiven Komponenten verringert wird oder diese Komponenten ihre Wirkung verlieren.

Aus diesem Grunde müssen solche Komponenten geschützt werden.

Die Erfindung kann für verschiedenste Applikationen eingesetzt werden. So können die erfindungsgemäßen Pellets durch entsprechende Auswahl geeigneter aktiver Komponenten als Nahrungsmittel für Tier und Mensch als Darreichungsform von Pharmazeutika, für Reinigungsmittelzusätze, als Pflanzenschutzmittel und anderes mehr eingesetzt werden.

So ist es aus dem Stand der Technik bekannt, ölige Komponenten, die zusätzlich auch aktive Komponenten enthalten können, zu emulgieren und eine solche Emulsion unmittelbar einzusetzen. Dies ist aber nur über einen begrenzten Zeitraum möglich, da die LangzeitStabilität solcher Emulsionen nicht unbegrenzt ist und außerdem Konservierungsstoffe häufig nicht eingesetzt werden können.

Aus diesem Grunde werden die Emulsionen zu Pulvern verarbeitet. Dies erfolgt, wie auch in EP 0 598 920 B1 beschrieben, durch Sprühtrocknung einer Emulsion. Gemäß der dort entnehmbaren Lehre wird eine Emulsion mit einer Ölphase und einer ganz bestimmten Sojabohnen-Hemi-Zellulose, die wasserlöslich ist, als Emulgiermittel hergestellt. Aus dieser Emulsion wird durch Sprühtrocknung ein Pulver in Form von Mikrokapseln hergestellt, dabei können in den Mikrokapseln neben der öligen Phase auch andere bzw. weitere aktive Komponenten enthalten sein.

Ein solches Pulver, das aus entsprechenden Mikrokapseln gebildet ist, erreicht aber eine begrenzte Beladung, hat also einen geringen Anteil an der jeweiligen aktiven Komponente, da die äußere Hülle ausreichend dick und dicht sein muss, um eine Beeinflussung durch Umweltfeuchtigkeit oder die Verflüchtigung von aktiven Komponenten aus den Mikrokapseln zu verhindern.

Des Weiteren ist verfahrensbedingt bei der Pulverherstellung durch Sprühtrocknung eine unerwünschte überwiegend thermisch bedingte negative Beeinflussung der aktiven Komponenten nicht vermeidbar, so dass zumindest ihre Wirksamkeit reduziert ist.

Dieser Sachverhalt trifft aber auch auf die in EP 1 214 892 A1 beschriebene Lösung zu. Hierbei ist es aber zusätzlich nachteilig, dass die jeweilige aktive Komponente, sei es ausschließlich eine ölige Komponente oder eine ölige Komponente zusätzlich mit einer weiteren aktiven Komponente, dass diese um einen inerten Kern verkapselt werden, so dass sich dementsprechend der in solchen Kapseln enthaltene Anteil an wirksamen Komponenten pro Volumen und in Bezug auf die Gesamtmasse reduziert ist.

Außerdem haben die herkömmlichen aus Emulsionen hergestellten Pulver eine begrenzte mechanische Festigkeit, so dass insbesondere bei Transport und Lagerung ein hoher Abrieb auftritt oder es gar zum Bruch der Kapseln und demzufolge zur Verflüchtigung oder unerwünschten Beeinflussung der Komponenten kommen kann.

Die durch Sprühtrocknung hergestellten Pulver sind nur bedingt fließfähig, so dass eine genaue Dosierung nur mit hohem Aufwand erreicht werden kann.

Sollen die Pulverpartikel zur Verbesserung der o.g. Eigenschaften mit einer äußeren Schützhülle (coating) überzogen werden, ist wegen ihrer sehr großen Oberflächen eine sehr große Menge an Beschichtungsstoff erforderlich. So können für die Ausbildung dichter Überzüge Masseverhältnisse von Pulver und Beschichtungsstoff bis zu 1:1 erforderlich sein, was zu erhöhten Herstellungskosten und einer Reduzierung des Anteils von aktiver Komponente zum Volumen der Masse führt.

Aus EP 1 252 831 A2 sind Partikel für Tabakprodukte bekannt, bei denen ein bestimmter Klang und Geschmack, die natürlichen Stoffen entsprechen, während des Rauchens erreicht werden sollen.

Es ist daher Aufgabe der Erfindung, oxidationsempfindliche zusätzlich oder allein auch leicht flüchtige aktive Komponenten in Form von Ölen und/oder in solchen Ölen zusätzlich enthaltenen aktiven Komponenten mit erhöhter Konzentration langzeitstabil für nachfolgende Verabreichung, Darreichung oder Weiterverarbeitung zur Verfügung zu stellen.

Erfindungsgemäß wird diese Aufgabe mit Pellets, die die Merkmale des Anspruchs 1 aufweisen, gelöst. Die erfindungsgemäßen Pellets können mit einem Verfahren gemäß Patentanspruch 23 hergestellt werden. Vorteilhafte Weiterbildungen und Ausgestaltungen der Erfindung können mit den in den untergeordneten Ansprüchen enthaltenen Merkmalen erreicht werden.

Gegenüber den aus dem Stand der Technik bekannten Lösungen zeichnen sich die erfindungsgemäßen Pellets dadurch aus, dass in ihnen die jeweiligen aktiven Komponenten in hoher Konzentration und trotzdem sicher geschützt enthalten sind.

Hierzu sind die aktiven Komponenten in homogener diskreter Verteilung in einer Matrix, die Hemi-Zellulose als Filmbildner enthält, verkapselt. Dabei sind in den einzelnen Pellets keinerlei inerte Kerne enthalten. Sie weisen jeweils eine Teilgröße von mindestens 100 µm, bevorzugt von mindestens 300 µm auf.

Der hohe Anteil an in solchen Pellets enthaltenen aktiven Komponenten kann insbesondere durch das ebenfalls erfindungsgemäße Herstellungsverfahren, wobei hierzu nachfolgend noch nähere Erläuterungen erfolgen, erreicht werden.

Das erfindungsgemäße einzusetzende Hemi-Zellulose als filmbildende Polysaccharid sollte mindestens einem Anteil von 0,5 Masse-% enthalten sein. Der Anteil dieser Filmbildner kann aber auch deutlich größer gewählt werden und bis zu 60 Masse-% betragen. Insbesondere der eingesetzte Filmbildner sichert, dass die eine gegebenenfalls auch mehrere eingeschlossene Komponente(n) in der Matrix in Form diskret angeordneter Kapseln eingeschlossen werden kann.

Wasserlösliche Hemi-Zellulose kann beispielsweise aus Sojabohnen oder Rapssamen gewonnen werden, und ist physiologisch und für die Umwelt unbedenklich.

Außerdem könnte sie gegenüber reinen Naturprodukten, wie z.B. Gummi arabicum in nahezu gleicher Qualität und mit nahezu konstanten Kosten, also unabhängig von witterungsbedingungen, die die Preise natürlicher filmbildenden Stoffe beeinflussen, zur Verfügung gestellt werden.

Des Weiteren ist eine deutlich höhere Verbraucherakzeptanz gegenüber der ebenfalls üblicherweise hierfür eingesetzten Gelatine gegeben.

Für die Matrix können nicht in Wasser lösliche Kohlenhydrate eingesetzt werden. Diese können aus Stärken oder Zellulose-Komponenten ausgewählt werden.

Es können unterschiedliche Stärken für die Ausbildung der Matrix eingesetzt werden, wobei hierfür besonders geeignet Malzstärke aber auch Getreidestärke ist. Diese können jeweils allein aber auch in Kombination miteinander eingesetzt werden.

Für die Matrixbildung eignet sich, als eine Zellulose-Komponente insbesondere mikrokristalline Zellulose (MCC).

Der Masseanteil an Stärke kann bis maximal 90 Masse-% gehalten sein.

Es ist aber auch möglich erfindungsgemäße Pellets mit wasserlöslichen Kohlehydraten herzustellen. So können Pellets in einem kontinuierlichen Spraygranulationsprozess hergestellt werden, in den solche Kohlehydrate in der Matrix enthalten sind und wasserlösliche Polysaccharide als Filmbildner fungieren.

Mit wasserlöslichen Kohlehydraten können die erreichbare Größe von Pellets in weiten Grenzen beeinflusst werden. So können eine Partikelgröße solcher Pellets im Bereich zwischen 200 µm bis 1500 µm erhalten werden. Es kann eine aktive Komponente mit mehr als 15 Masse-% verkapselt werden.

Hierfür geeignete Kohlehydrate sind unterschiedliche Oligosaccharide mit unterschiedlicher Kettenlänge. Besonders geeignet sind hierfür Dextrine und ganz besonders geeignet sind Maltosedextrine.

Neben den genannten organischen Komponenten kann eine Matrix auch aus anorganischen Komponenten gebildet sein oder solche Komponenten enthalten.

Geeignete anorganische Stoffe sind beispielsweise Kaolin, CaCO₃, CaS, Silikate, Ton, Bentonit, Diatromeein Erde oder Aluminiumoxid, die auch als Mischung eingesetzt werden können.

Auch die anorganischen Komponenten sollten weitestgehend nicht in Wasser löslich sein.

Des Weiteren können aber auch Mono-, Di- oder Trisaccharide enthalten sein, wobei auch hier unterschiedliche Masseanteile, die oberhalb 50 Masse-% liegen können, eingehalten werden können.

Neben neutralen öligen Komponenten, also solche, die keine aktive Wirkung ausüben, können aber auch ölige Komponenten mit aktiver Wirkung für die Herstellung erfindungsgemäßer Pellets eingesetzt werden. Der Anteil dieser Komponente sollte bevorzugt mindestens 15 Masse-% betragen. Hierfür haben sich insbesondere die verschiedenen Fruchtöle aber auch Fruchtölextrakte als vorteilhaft herausgestellt. So kann die ölige Komponente beispielsweise aus Orangen- und/oder Zitronenöl gebildet sein. Dabei kann bereits die aromatische Geschmacks- und Geruchsnote dieser öligen Komponenten die gewünschte aktive Wirkung allein gegebenenfalls aber auch mit zusätzlichen aktiven Komponenten erzielen.

Als zusätzliche aktive Komponente können aber auch synthetische oder natürliche Farbstoffe eingesetzt werden. So kann beispielsweise Karotin, bevorzugt Beta Karotin, Kantaxanthin oder Astaxanthin, die neben der Farbstoffwirkung die einschlägig bekannten zusätzlichen Wirkungen aufweisen, hierfür eingesetzt werden.

Weiter können als aktive Komponenten aber auch Vitamine, bevorzugt in Öl lösliche Vitamine, wie z.B. Vitamin A Acetat als aktive Komponente eingesetzt werden, wobei auch eine Kombination von diesen Vitaminen mit Karotin möglich ist.

Neben den Vitaminen können aber auch andere pharmazeutisch wirksame Stoffe als aktive Komponente eingesetzt werden. Weitere möglich aktive Komponenten sind verschiedene Insektizide/Biozide.

Für nachfolgende Anwendungen der erfindungsgemäßen Pellets in Reinigungsmitteln aber auch gegebenenfalls in Kosmetika können unterschiedliche Aromastoffe mit entsprechend angenehmer Duftnote gegebenenfalls zusätzlich mit Tensiden eingesetzt werden.

Für Nahrungsmittel können Aromastoffe mit Geschmacks- und/oder Duftnote eingesetzt werden.

Als aktive Komponente können aber auch ungesättigte Fettsäuren, wie z.B. Alpha-Omega-Polyungesättigte Fettsäuren eingesetzt werden.

Zusätzlich in den Kapseln neben der öligen Komponente enthaltene aktive Komponenten muss nicht zwingend Öl löslich sein. Sie können auch in dispergierter Form, als kleine Kristalle in einer öligen Komponente enthalten sein. So besteht die Möglichkeit, instabile Vitamine, wie z.B. das Vitamin K (MSBC) feinkristallin in einer öligen Komponente zu dispergieren und in den Pellets entsprechend mit der öligen Komponente zu verkapseln.

Der Anteil der von den in der Matrix eingebetteten Kapseln eingenommenem Volumen sollte oberhalb 10%, bevorzugt oberhalb 20% gehalten werden. Es besteht aber auch die Möglichkeit den entsprechenden Volumenanteil darüber hinausgehend einzustellen und dementsprechend den Anteil, also die Beladung mit aktiven Komponenten in den Pellets deutlich zu erhöhen.

Der Wasseranteil in den fertigen Pellets sollte kleiner als 10 Masse-% gehalten werden.

Die erfindungsgemäßen Pellets werden im Gegensatz zu den aus dem Stand der Technik durch Sprühtrocknung hergestellten Pulvern durch eine Kombination von E-mulsionsherstellung mit nachfolgender Direktpelletierung oder Extrusion erhalten.

Dabei werden für die Herstellung der Emulsion mindestens eine Stärke, eine ölige Komponente, wasserlösliches Hemi-Zellulose als Filmbildner und Wasser eingesetzt.

Der daraus erhaltenen Emulsion kann zusätzlich beispielsweise Stärke zugegeben werden, um die Viskosität zu erhöhen und eine teigige Konsistenz einzustellen.

In diesem Zustand kann bevorzugt eine Extrusion erfolgen, wobei aus dem jeweiligen Extruder dann die noch leicht feuchten Pellets entnommen werden können. Die Pellets können gegebenenfalls noch mechanisch nachbearbeitet, bevorzugt verrundet werden.

Es kann sich außerdem eine Nachtrocknung anschließen, um den Wassergehalt weiter zu reduzieren und es besteht die Möglichkeit auch die einzelnen Pellets mit einer schützenden Beschichtung zu versehen, die bevorzugt einen Feuchtigkeitsschutz bildet und gegebenenfalls auch ein miteinander Verkleben der Pellets verhindern kann.

Es hat sich in überraschender Weise herausgestellt, dass eine Emulsion, in der eine ölige Komponente zumindest fein dispergiert verteilt ist und mit wasserlöslichen Hemi-Zellulose als Filmbildner durch z.B. Zugabe viskositätserhöhender weiterer Stärke bei einer Extrusion die gebildeten, die ölige Komponente und gegebenenfalls weitere aktive Komponenten enthaltenden Kapseln in die Matrix eingebettet und dabei nicht zerstört werden. Außerdem kann eine homogene, also eine sehr gleichmäßige Verteilung der kleinen Kapseln innerhalb der Matrix der einzelnen Pellets erreicht werden. Je nach Anzahl und dementsprechend dem Anteil der in die Matrix eingebetteten Kapseln kann eine mehr oder weniger große Beladung, also ein entsprechender Anteil an aktiven Komponenten eingestellt werden, der bis hin zu 20 Masse-% und auch darüber hinaus gehalten werden kann.

Beim Extrudieren können auch relativ feine Düsen genutzt werden, so dass die Pellets nach dem Extrudieren orthogonal zur Extrusionsrichtung Durchmesser im Bereich um 1 mm aufweisen können.

Selbstverständlich sind auch größere und gegebenenfalls auch etwas unter 1 mm liegende Pelletquerschnitte erreichbar.

Bei der Extrusion kann bei insbesondere gegenüber der Sprühtrocknung erniedrigter Temperatur gearbeitet werden, so dass die eingesetzten aktiven Komponenten dadurch ebenfalls geringer negativ beeinflusst werden. Es treten weniger Verdampfungsverluste auf.

Bei der Extrusion kann mit relativ niedrigen Drücken gearbeitet werden. Auch hierdurch kann eine schonende Behandlung der verkapselten aktiven Komponenten erreicht und gesichert werden, dass die aktiven Komponenten vor den unerwünschten äußeren Einflüssen geschützt und sicher innerhalb der Matrix der Pellets eingeschlossen gehalten werden können.

Die im Wesentlichen für die Ausbildung der Matrix eingesetzte Stärke und/oder Zellulose-Komponente ist physiologisch und für die Umwelt unbedenklich und überwiegend auch geruchs- und geschmacksneutral.

Außerdem weisen die erfindungsgemäß hergestellten Pellets auch eine gute mechanische Stabilität auf, so dass ein reduzierter Abrieb erreichbar ist.

Nachfolgend soll die Erfindung beispielhaft näher beschrieben werden.

### Beispiel 1 (Vergleich)

Für die Herstellung einer Emulsion wurden 10 kg modifizierte Stärke (Hicap) mit 30 kg Wasser, 8 kg Orangenöl und 2 kg Malzstärke intensiv vermischt. Im Anschluss daran erfolgte eine Homogenisierung bei ca. 200 bar in einem Homogenisierer der Firma Niro. Die so vorbereitete Emulsion wurde durch Zugabe von 12 kg Getreidestärkepulver mit anschließender Vermischung in eine teigartige Konsistenz gebracht. Die so erhaltene Masse wird dann mit einem Niedrigdruckextruder Fuji-Paudal extrudiert. Dabei wurde ein Pelletquerschnitt von 1,2 mm mit entsprechenden Extruderdüsen eingestellt.

Die so erhaltenen Pellets wurden anschließend verrundet und in einem Wirbelbett nachfolgend nachgetrocknet, bis ein Wasseranteil von etwa 4% eingehalten werden konnte.

Der Verlust an Orangenöl, als aktiver Komponente lag bei 8%, so dass der Masseanteil an Orangenöl in den fertigen Pellets oberhalb 18 Masse-% gehalten werden konnte.

### Beispiel 2

Für die Herstellung der Emulsion wurden 6 kg Hemi-Zellulose, als Filmbildner auf Sojabohnenbasis, 6 kg Malzstärke, 30 kg Wasser und 36 kg Orangenöl intensivst miteinander vermischt. Diese Präemulsion wurde nachfolgend ebenfalls bei ca. 200 bar in einem Homogenisierer der Firma Niro homogenisiert.

Dieser homogenisierten Emulsion wurden anschließend 6 kg Malzstärke und 9 kg Getreidestärke zugegeben und so miteinander vermischt, dass eine teigartige, extrudierbare Konsistenz eingestellt worden ist. Diese wurde dann ebenfalls, wie beim Beispiel 1 bereits erläutert, extrudiert.

Der Wassergehalt lag ebenfalls nach erfolgter Trocknung bei ca. 4 Masse-%, der Anteil an Orangenöl konnte auf 54 Masse-% eingestellt werden, wobei lediglich ein Verlust von 2% an Orangenöl zu verzeichnen war.

### Beispiel 3 (Vergleich)

Hierbei wurden 16 kg modifizierte Stärke (Hicap 100), 27,2 kg Wasser, 6,86 kg Vitamin A Acetatöl bei einer Temperatur von 60 °C intensiv vermischt und anschließend wie bei den Beispielen mit einer nachfolgenden Homogenisierung die fertige Emulsion erhalten. Dieser Emulsion wurden 2,5 kg Malzstärke und 7,6 kg Getreidestärke zugegeben und nachfolgend erfolgte wiederum eine Extrusion mit sich daran anschließender Verrundung und Nachtrocknung auf einen Wassergehalt von ca. 4 Masse-%.

### Beispiel 4

Für die Herstellung einer Emulsion wurden 6 kg Hemi-Zellulose, auf Sojabohnenbasis, 12 kg Malzstärke, 30 kg Wasser mit 36 kg nichtanionischer Säure bei Temperaturen zwischen 0 und 5 °C intensiv miteinander vermischt und nachfolgen ebenfalls homogenisiert.

Die teigartige Konsistenz wurde durch Zugabe von 9 kg Getreidestärke eingestellt und es erfolgte nachfolgend wiederum eine Verrundung und Nachtrocknung auf einen Wasseranteil von ca. 4 Masse-%.

Die nichtanionische Säure war in den Pellets sicher verkapselt und es konnte kein Schwund festgestellt werden.

### Beispiel 5

Dabei wurden 125 g pulverförmige Malzstärke mit 125 g Hemi-Zellulose auf Sojabohnenbasis in Pulverform vermischt. Diese Pulvermischung wurde in 1900 g entmineralisiertem Wasser gelöst und vermischt. Dieser Lösung wurden 1350 g eines Öles mit einem hohen Anteil polyungesättigter-Fettsäure-Esther zugegeben und intensivst vermischt. Das Öl hatte eine Jodzahl von 170. Diese voremulgierte Mischung wurde mit einem Zweistufenhomogenisierer der Firma Niro bei Drücken von 200 bar und 220 bar homogenisiert und weiter emulgiert.

Der größte Teil der Oltropfen in der so erhaltenen Emulsion hatte eine Partikelgröße von 1,4 µm.

Nachfolgend wurde eine Mischung von 1289 g einer Maisstärke (Cerestar) und 1289 g mikrokristalliner Zellulose der Emulsion, die eine Masse von 2380 g aufwies sowie zusätzlich 496 g Wasser zugegeben und miteinander vermischt. So konnte eine teigartige Konsistenz mit einem Feuchtigkeitsanteil von 36 Masse-% erreicht werden.

Diese teigartige Masse wurde mit einem Extruder Fuji-Paudal mit geringem Druck extrudiert, wobei die Extruderdüsen einen Durchmesser von 0,8 mm aufwiesen.

Nachfolgend wurden die extrudierten Pellets in einem Wirbelbett auf einen Wasseranteil von 3 Masse-% getrocknet.
Die getrockneten Pellets wiesen einen Ölgehalt von 25 Masse-% auf, der über einen Zeitraum von vier Wochen bei Raumtemperatur und offener Lagerung bei atmosphärischen Bedingungen überwacht wurde. Nach diesen vier Wochen konnte eine Jodzahl von 162 ermittelt werden. Dies zeigte, dass nahezu keine Oxidation bei der Herstellung und nachfolgenden Lagerung zu verzeichnen war. Der freie Fettanteil konnte unterhalb von 0,1 Masse-% ermittelt werden.

### Beispiel 6

350 g einer pulverförmigen Hemi-Zellulose auf Sojabohnenbasis wurden mit 700 g Malzstärke vermischt und in 2450 g entmineralisiertem Wasser gelöst. Dieser Lösung wurden 1500 g Orangenöl zugegeben und es erfolgte eine intensivste Vermischung. Die Mischung wurde bei einer Temperatur unterhalb 10 °C gehalten.

Die voremulgierte Mischung wurde mit einem Niro Zweistufenhomogenisierer bei Drücken von 200 bar und 220 bar homogenisiert. Der größte Anteil der in der Emulsion enthaltenen Öltropfen hatte eine Partikelgröße von 0,8 µm.

3950 g dieser Emulsion wurden 950 g mikrokristalline Zellulose (Vivapur) sowie 100 g Weizenstärke zugegeben und miteinander vermischt, so dass eine teigartige Konsistenz mit einem Feuchtigkeitsanteil von 39 Masse-% erreicht wurde. Nachfolgend erfolgten eine Extrusion und daran nachfolgend eine Sphäronisierung und Trocknung in einem Wirbelbett.

Die getrockneten Pellets wurden bei 40 °C und einer relativen Luftfeuchtigkeit von 35 % offen unter Lufteinfluss gelagert. Zu Beginn und am Ende der Lagerung wurde der Anteil an Cis- und Translimonenepoxid, Carveole und Carvone bestimmt. Die erhaltenen Messwerte sind in der nachfolgenden Tabelle 1 aufgezeigt.

**Tabelle 1**

| Oxidations Komponente | Konzentration der oxidierenden Komponente in ppm Orangenöl bei Beginn | Konzentration der oxidierenden Komponente in ppm Orangenöl nach 3 Monaten bei 40 °C/ 35 relative Luftfeuchtigkeit |
|---|---|---|
| | | |
| Cis-Limonenepoxid | 225 ppm | 2000 ppm |
| | | |
| Trans-Limonenepoxid | 150 ppm | 1100 ppm |
| | | |
| Carveole | 75 ppm | 1300 ppm |
| | | |
| Carvone | <10 ppm | 900 ppm |

### Beispiel 7 (Vergleich)

Der extrudierbaren Masse mit teigartiger Konsistenz, die nach dem Beispiel 3
erhalten wurde, wurde mit einer Masse von 10 kg in einen Granulierer der Firma Glatt und zusätzlich 1 kg Maisstärke Cerestar gegeben.

Die Granulierung wurde über einen Zeitraum von fünf Minuten durchgeführt und das so erhaltene Granulat einem Sphäronisierer ebenfalls von der Firma Glatt zugeführt. Die sphäronisierten Pellets wurden nachfolgend in einem Wirbelbett nachgetrocknet, bis ein Feuchtigkeitsanteil von 3 Masse-% erreicht worden ist.

### Beispiel 8

Hierbei wurden 0,5 kg Zellulose auf Sojabohnenbasis mit 2 kg Maltrosedextrin vermischt und langsam in 12,5 kg demineralisiertem Wasser gelöst.

7,5 kg Maltosedextrin der gleichen Qualität wurden dieser Mischung gemeinsam mit 3 kg polyungesättigtem Öl, das von C.N. Schmidt, B. V. Amsterdam, Niederlande erhältlich ist und eine Jodzahl von 170 aufwies, zugegeben und mit einem Hochgeschwindigkeitsmischer zu einer 22,5 kg aufweisenden Präemulsion verarbeitet. Diese Präemulsion wurde mit einem Zweistufenhochdruckhomogenisierer bei einem Druck von 250 bis 275 bar homogenisiert.

Diese Mischung wurde in einem Fluidbett einer WFA 225 Direktpelletisierereinrichtung, die von der Firma Glatt GmbH hergestellt worden ist, gegeben. Die so erhaltenen Produkte wurden im Anschluss an eine Lagerung an Luftatmosphäre über einen Zeitraum von 4 Wochen beobachtet, wobei der Anteil von Hexanal als O-xidationsprodukt weniger als 20 % angestiegen ist.

Die einzelnen Pellets hatten eine Partikelgröße im Bereich zwischen 250 bis 355 µm.

Es ist aber in gleicher Form möglich Pellets mit einer Partikelgröße im Bereich zwischen 800 bis 1118 µm mit der gleichen Rezeptur herzustellen.

Alle so hergestellten Pellets hatten einen Anteil an enthaltenem Öl von mehr als 23 Masse-% und einen Hexanalanteil ≤ 0,5 mg/kg in Bezug zum Öl.

### Beispiel 8a

Mit der gleichen Rezeptur, wie beim Beispiel 8 wurden Pellets erfindungsgemäß hergestellt, in denen Orangenöl verkapselt wurde. Anstelle der 3 kg ungesättigten Öles wurden 2 kg Orangenöl zugegeben, das einen Limonenanteil von 63 % aufwies. Diese Mischung wurde homogenisiert, wie dies auch beim Beispiel 8 der Fall war.

Die so erhaltene Mischung wurde in einem Fluidbett einer Direktpelletieranlage WSA 225, der Firma Glatt GmbH verarbeitet. Die dabei erhaltenen Pellets hatten im Wesentlichen eine Partikelgröße zwischen 800 bis 1100 µm und einen Anteil an Limonen von 19 Masse-%. Der Verlust an Orangenöl während der Direktpelletierung betrug weniger als 5 % und es war keine Oxidation von Limonen während des Pelletisier- und Trocknungsprozesses zu erkennen.

### Beispiel 9

125 g einer pulverförmigen Hemi-Zellulose auf Sojabohnenbasis wurden in Mischung mit 125 g Malzstärke in 1250 g entmineralisiertem Wasser gelöst.

Dieser Lösung wurden 1250 g Arachidon-Säure zugegeben und intensivst vermischt.

Die voremulgierte Mischung wurde in zwei Stufen bei Drücken von 200 bar und 350 bar homogenisiert. Die einzelnen Ölemulsionspartikel hatten im Wesentlichen eine Größe von 0,8 µm.

Anschließend wurden 645 g dieser Emulsion mit 2000 g Kaolin (GTY Clay) und 600 g mikrokristalline Zellulose vermischt und eine extrudierbare teigartige Konsistenz mit einem Wasseranteil von 28 Masse-% erreicht.

Nachfolgend wurde die Masse extrudiert, eine Sphäronisierung und eine Trocknung im Wirbelbett durchgeführt, bis ein Wasseranteil von 3 Masse-% erreicht worden ist.

Der Anteil an verkapselter Arachidon-Säure lag bei 8,5 Masse-%.

Bei einer Lagerung an Luft bei Raumtemperatur konnten keine Verluste oder eine Beeinträchtigung der Arachidon-Säure festgestellt werden.

## Patentansprüche

1. Pellet, in dem in homogener diskreter Verteilung mindestens eine ölige oxidationsempfindliche und/oder leicht flüchtige aktive Komponente in einer Matrix, die Hemi-Zellulose als Filmbildner enthält, kernlos verkapselt ist, dabei die Kapseln, in denen die Komponente (n) eingeschlossen ist/sind, in der Matrix diskret angeordnet sind; und es jeweils eine Teilgröße von mindestens 100 µm aufweist.

2. Pellet nach Anspruch 1,
**dadurch gekennzeichnet, dass** Hemi-Zellulose als wasserlösliches filmbildendes Polysaccharid mit mindestens 0,5 Masse-% enthalten ist.

3. Pellet nach Anspruch 1 oder 2,
**dadurch gekennzeichnet, dass** die ölige oxidationsempfindliche Komponente mit mindestens 15 Masse-% enthalten ist.

4. Pellet nach einem der vorhergehenden Ansprüche,
**dadurch gekennzeichnet, dass** es eine Teilgröße von mindestens 300 µm aufweist.

5. Pellet nach einem der vorhergehenden Ansprüche,
**dadurch gekennzeichnet, dass** in der Matrix mindestens ein nicht wasserlösliches Kohlenhydrat enthalten ist.

6. Pellet nach Anspruch 5,
**dadurch gekennzeichnet, dass** das nicht wasserlösliche Kohlenhydrat eine Stärke und/oder eine Zellulose-Komponente ist.

7. Pellet nach Anspruch 6,
**dadurch gekennzeichnet, dass** das nicht wasserlösliche Kohlenhydrat eine Getreidestärke, mikrokristalline Zellulose, Malzstärke oder ein Gemisch davon ist.

8. Pellet nach einem der vorhergehenden Ansprüche,
**dadurch gekennzeichnet, dass** in der Matrix Mono-, Di- oder Tri-Saccharide enthalten sind.

9. Pellet nach einem der vorhergehenden Ansprüche,
**dadurch gekennzeichnet, dass** in der Matrix eine anorganische Komponente, die in Wasser nicht löslich ist, enthalten ist.

10. Pellet nach einem der vorhergehenden Ansprüche,
**dadurch gekennzeichnet, dass** die anorganische Komponente Kaolin, CaCO₃, CaS, ein Silikat, Aluminiumoxid, Ton, Bentonit oder Diatomeein Erde ist.

11. Pellet nach einem der vorhergehenden Ansprüche,
**dadurch gekennzeichnet, dass** mindestens ein wasserlösliches Kohlenhydrat in der Matrix enthalten ist.

12. Pellet nach Anspruch 11,
**dadurch gekennzeichnet, dass** das mindestens eine wasserlösliches Kohlenhydrat aus der Gruppe der Dextrine und Maltosedextrine ausgewählt ist.

13. Pellet nach Anspruch 11 oder 12,
**dadurch gekennzeichnet, dass** die Partikelgröße von Pellets im Bereich 200 bis 1500 µm liegt.

14. Pellet nach einem der vorhergehenden Ansprüche,
**dadurch gekennzeichnet, dass** die ölige Komponente ein aromatisches Fruchtöl oder ein solcher Fruchtölextrakt ist.

15. Pellet nach einem der vorhergehenden Ansprüche,
**dadurch gekennzeichnet, dass** die aktive Komponente ein Farbstoff ist.

16. Pellet nach einem der vorhergehenden Ansprüche,
**dadurch gekennzeichnet, dass** die aktive Komponente ein Aromastoff ist.

17. Pellet nach einem der vorhergehenden Ansprüche,
**dadurch gekennzeichnet, dass** die aktive Komponente eine ungesättigte Fettsäure ist.

18. Pellet nach einem der vorhergehenden Ansprüche,
**dadurch gekennzeichnet, dass** die aktive Komponente ein pharmazeutischer Wirkstoff ist oder einen pharmazeutischen Wirkstoff enthält.

19. Pellet nach einem der vorhergehenden Ansprüche,
**dadurch gekennzeichnet, dass** die aktive Komponente ein Insektizid/Biozid ist.

20. Pellet nach einem der vorhergehenden Ansprüche,
**dadurch gekennzeichnet, dass** die aktive Komponente in einer öligen Komponente löslich oder dispergierbar ist.

21. Pellet nach einem der vorhergehenden Ansprüche,
**dadurch gekennzeichnet, dass** die Kapseln ein Volumen von mindestens 10 % im Pellet einnehmen.

22. Pellet nach einem der vorhergehenden Ansprüche,
**dadurch gekennzeichnet, dass** der Wassergehalt kleiner als 10 Masse-% ist.

23. Verfahren zur Herstellung von Pellets, in denen mindestens eine ölige Komponente allein oder mit einer zusätzlichen aktiven Komponente kernlos in verkapselter Form in einer Matrix enthalten ist und einzelne Kapseln, in denen die Komponente(n) eingeschlosen ist/sind, diskret und homogen verteilt angeordnet sind;
bei dem eine wässrige Emulsion, die neben mindestens einer öligen Komponente, Hemi-Zellulose als ein filmbildendes, wasserlösliches Polysaccharid enthält, in der die ölige Komponente in feinverteilter Form enthalten ist, hergestellt wird,
dieser Emulsion ein Matrix bildender Stoff oder Stoffgemisch zur Einstellung einer teigigen Konsistenz zugegeben und daraus die Pellets hergestellt werden.

24. Verfahren nach Anspruch 23,
**dadurch gekennzeichnet, dass** die Pellets durch Extrusion oder Direktpelletierung hergestellt - werden.

25. Verfahren nach einem der Ansprüche 23 oder 24,
**dadurch gekennzeichnet, dass** die Emulsion in zwei Stufen hergestellt wird, wobei bei einer zweiten Stufe eine Homogenisierung bei erhöhtem Druck durchgeführt wird.

26. Verfahren nach einem der Ansprüche 23 bis 25,
**dadurch gekennzeichnet, dass** im Anschluss an die Extrusion eine Verrundung der Pellets durchgeführt wird.

27. Verfahren nach einem der Ansprüche 23 bis 26,
**dadurch gekennzeichnet, dass** die Pellets nachgetrocknet werden.

28. Verfahren nach einem der Ansprüche 23 bis 27,
**dadurch gekennzeichnet, dass** die Pellets mit einer schützenden Beschichtung versehen werden.

## Claims

1. A pellet, in which at least one oily oxidation-sensitive and/or highly volatile active component is, in homogeneous discrete distribution, corelessly encapsulated in a matrix containing hemicellulose as film former, with the capsules in which the component or components is or are enclosed being arranged discretely in the matrix, and each pellet having a fraction size of at least 100 µm.

2. A pellet according to claim 1,
**characterised in that** hemicellulose is present as a water-soluble film-forming polysaccharide at least at 0.5% w/w.

3. A pellet according to claim 1 or 2,
**characterised in that** the oily oxidation-sensitive component is present at least at 15% w/w.

4. A pellet according to any one of the preceding claims,
**characterised in that** the pellet has a fraction size of at least 300 µm.

5. A pellet according to any one of the preceding claims,
**characterised in that** the matrix contains at least one non-water-soluble carbohydrate.

6. A pellet according to claim 5,
**characterised in that** the non-water-soluble carbohydrate is a starch and/or a cellulose component.

7. A pellet according to claim 6,
**characterised in that** the non-water-soluble carbohydrate is a cereal starch, microcrystalline cellulose, malt starch or a mixture thereof

8. A pellet according to any one of the preceding claims,
**characterised in that** the matrix contains mono-, di- or trisaccharides.

9. A pellet according to any one of the preceding claims,
**characterised in that** the matrix contains an inorganic component which is not soluble in water.

10. A pellet according to any one of the preceding claims,
**characterised in that** the inorganic component is kaolin, CaCO₃, CaS, a silicate, aluminium oxide, clay, bentonite or diatomaceous earth.

11. A pellet according to any one of the preceding claims,
**characterised in that** the matrix contains at least one water-soluble carbohydrate.

12. A pellet according to claim 11,
**characterised in that** the at least one water-soluble carbohydrate is selected from the group consisting of dextrins and maltodextrins.

13. A pellet according to claim 11 or 12,
**characterised in that** the particle size of the pellets is in the range 200 to 1500 µm.

14. A pellet according to any one of the preceding claims,
**characterised in that** the oily component is an aromatic fruit oil or a fruit oil extract of such kind.

15. A pellet according to any one of the preceding claims,
**characterised in that** the active component is a colour.

16. A pellet according to any one of the preceding claims,
**characterised in that** the active component is an aromatic substance.

17. A pellet according to any one of the preceding claims,
**characterised in that** the active component is an unsaturated fatty acid.

18. A pellet according to any one of the preceding claims,
**characterised in that** the active component is a pharmaceutical active ingredient or contains a pharmaceutical active ingredient.

19. A pellet according to any one of the preceding claims,
**characterised in that** the active component is an insecticide/biocide.

20. A pellet according to any one of the preceding claims,
**characterised in that** the active component is soluble or dispersible in an oily component.

21. A pellet according to any one of the preceding claims,
**characterised in that** the capsules occupy a volume of at least 10% in the pellet.

22. A pellet according to any one of the preceding claims,
**characterised in that** the water content is less than 10% w/w.

23. A process for producing pellets in which at least one oily component is, either on its own or with an additional active component, corelessly contained in encapsulated form in a matrix, and individual capsules in which the component or components is or are enclosed are arranged in a discretely and homogeneously distributed manner;
in which process there is produced an aqueous emulsion which, in addition to at least one oily component, contains hemicellulose as a film-forming water-soluble polysaccharide, and in which emulsion the oily component is present in finely dispersed form,
to this emulsion is added a matrix-forming substance or mixture of substances for setting a doughy consistency, and the pellets are produced therefrom.

24. A process according to claim 23,
**characterised in that** the pellets are produced by extrusion or direct pelleting.

25. A process according to one of claims 23 or 24,
**characterised in that** the emulsion is produced in two stages, with homogenisation being carried out at elevated pressure in a second stage.

26. A process according to any one of claims 23 to 25,
**characterised in that,** following extrusion, the pellets are rounded.

27. A process according to any one of claims 23 to 26,
**characterised in that** the pellets are after-dried.

28. A process according to any one of claims 23 to 27,
**characterised in that** the pellets are provided with a protective coating.

## Revendications

1. Granulé, dans lequel au moins un composant actif huileux sensible à l'oxydation et/ou légèrement volatil est encapsulé sous une forme exempte de noyau, selon une distribution homogène discrète, dans une matrice qui contient de l'hémicellulose comme agent filmogène, les capsules obtenues de la sorte, dans lesquelles le ou les composants sont enfermés, étant agencées de manière discrète dans la matrice ; et présentant, respectivement, une taille particulaire d'au moins 100 µm.

2. Granulé selon la revendication 1,
**caractérisé en ce qu'**il contient de l'hémicellulose sous la forme d'un polysaccharide filmogène hydrosoluble à raison d'au moins 0,5 % en masse.

3. Granulé selon la revendication 1 ou 2,
**caractérisé en qu'**il contient le composant huileux sensible à l'oxydation à raison d'au moins 15 % en masse.

4. Granulé selon l'une quelconque des revendications précédentes, **caractérisé en ce qu'**il présente une taille particulaire d'au moins 300 µm.

5. Granulé selon l'une quelconque des revendications précédentes, **caractérisé en ce qu'**il contient, dans la matrice, au moins un hydrate de carbone non hydrosoluble.

6. Granulé selon la revendication 5,
**caractérisé en ce que** l'hydrate de carbone non hydrosoluble est un composant à base d'amidon et/ou un composant à base de cellulose.

7. Granulé selon la revendication 6,
**caractérisé en ce que** l'hydrate de carbone non hydrosoluble est un amidon de céréales, une cellulose microcristalline, un amidon du malt ou un mélange de ceux-ci.

8. Granulé selon l'une quelconque des revendications précédentes, **caractérisé en ce que** la matrice contient des mono-, di- ou trisaccharides.

9. Granulé selon l'une quelconque des revendications précédentes, **caractérisé en ce que** la matrice contient un composant inorganique, qui n'est pas soluble dans l'eau.

10. Granulé selon l'une quelconque des revendications précédentes, **caractérisé en ce que** le composant inorganique est du kaolin, du CaCO₃, du CaS, un silicate, de l'oxyde d'aluminium, de l'argile, de la bentonite ou de la terre à diatomées.

11. Granulé selon l'une quelconque des revendications précédentes, **caractérisé en ce que** la matrice contient au moins un hydrate de carbone hydrosoluble.

12. Granulé selon la revendication 11,
**caractérisé en ce que** le au moins un hydrate de carbone est choisi dans le groupe constitué des dextrines et des dextrine-maltoses.

13. Granulé selon la revendication 11 ou 12,
**caractérisé en ce que** la taille particulaire des granulés se situe dans la plage de 200 à 1500 µm.

14. Granulé selon l'une quelconque des revendications précédentes, **caractérisé en ce que** le composant huileux est une huile aromatique de fruits ou ce type d'extrait d'huile de fruits.

15. Granulé selon l'une quelconque des revendications précédentes, **caractérisé en ce que** le composant actif est un colorant.

16. Granulé selon l'une quelconque des revendications précédentes, **caractérisé en ce que** le composant actif est une substance aromatisante.

17. Granulé selon l'une quelconque des revendications précédentes, **caractérisé en ce que** le composant actif est un acide gras insaturé.

18. Granulé selon l'une quelconque des revendications précédentes, **caractérisé en ce que** le composant actif est un agent pharmaceutique ou contient un agent pharmaceutique.

19. Granulé selon l'une quelconque des revendications précédentes, **caractérisé en ce que** le composant actif est un insecticide/biocide.

20. Granulé selon l'une quelconque des revendications précédentes, **caractérisé en ce que** le composant actif est soluble ou dispersable dans un composant huileux.

21. Granulé selon l'une quelconque des revendications précédentes, **caractérisé en ce que** les capsules occupent un volume d'au moins 10 % dans le granulé.

22. Granulé selon l'une quelconque des revendications précédentes, **caractérisé en ce que** la teneur en eau est inférieure à 10 % en masse.

23. Procédé de fabrication de granulés, dans lesquels au moins un composant huileux, seul ou conjointement avec un autre composant actif, est contenu sous une forme exempte de noyau et encapsulée, dans une matrice, les capsules individuelles obtenues de la sorte, dans lesquelles le ou les composants sont enfermés, étant agencées selon une distribution discrète et homogène ;
dans lequel on fabrique une émulsion aqueuse qui contient, en plus d'au moins un composant huileux, de l'hémicellulose sous la forme d'un polysaccharide filmogène hydrosoluble, dans laquelle le composant huileux est contenu sous une forme finement divisée,
on ajoute à cette émulsion une substance formant une matrice ou un mélange de substances pour régler la consistance pâteuse, et on fabrique les granulés à partir de celle-ci.

24. Procédé selon la revendication 23,
**caractérisé en ce que** les granulés sont fabriqués par extrusion ou par granulation directe.

25. Procédé selon la revendication 23 ou 24, **caractérisé en ce que** l'émulsion est préparée en deux étapes, une homogénéisation à pression élevée étant effectuée à la seconde étape.

26. Procédé selon l'une quelconque des revendications 23 à 25, **caractérisé en ce que** l'opération d'extrusion est suivie d'une opération de sphéroïdisation des granulés.

27. Procédé selon l'une quelconque des revendications 23 à 26, **caractérisé en ce que** les granulés sont ensuite séchés.

28. Procédé selon l'une quelconque des revendications 23 à 27, **caractérisé en ce que** les granulés sont pourvus d'un revêtement de protection.
